# EUROPEAN PATENT SPECIFICATION

(11) **EP 2 796 082 B1**
(45) Date of publication and mention of the grant of the patent: **19.10.2016**
(21) Application number: 12858769.8
(22) Date of filing: 17.12.2012
(51) Int. Cl.: A61B 1/00, A61B 1/06, G02B 23/26, F21K 99/00, G02B 23/24

(54) **ILLUMINATING UNIT FOR ENDOSCOPE, AND ENDOSCOPE**
BELEUCHTUNGSEINHEIT FÜR EIN ENDOSKOP UND ENDOSKOP
UNITÉ D'ÉCLAIRAGE POUR ENDOSCOPE ET ENDOSCOPE

(30) Priority: 19.12.2011 JP 2011277321
(43) Date of publication of application: 29.10.2014
(73) Proprietor: FUJIFILM Corporation, Tokyo 106-8620 (JP)
(72) Inventor: KOMUKAI, Makito, Ashigarakami-gun Kanagawa 258-8538 (JP)
(74) Representative: Klunker . Schmitt-Nilson . Hirsch
(86) International application number: PCT/JP2012/082699
(87) International publication number: WO 2013/094569

(56) References cited:
- EP-A1- 1 787 571
- JP-A- 2005 502 083
- JP-A- 2007 020 937
- JP-A- 2010 160 948
- JP-A- 2011 072 424
- US-B2- 7 878 696

## Description

### BACKGROUND OF THE INVENTION

### 1. Field of the Invention

The present invention relates to an illumination unit for an endoscope and an endoscope.

### 2. Description of the Related Art

In an endoscope apparatus including a medical endoscope that is used for observation, treatment, or the like inside a living body, an illumination window and an observation window are formed at a tip of an insertion section of an endoscope, illumination light is emitted from the illumination window, and an observed image is obtained through the observation window. Light emitted from a light source device, such as a xenon lamp, is guided to the illumination window by a light guide member such as an optical fiber bundle, and is emitted from the illumination window. In recent years, an endoscope apparatus, which uses a laser light source instead of the illumination light using the light source device and generates illumination light by making a fluorescent body disposed at a tip of the insertion section of the endoscope be excited and emit light, has been used (for example, JP2007-20937A and JP2011-72424A).

### SUMMARY OF THE INVENTION

Incidentally, since the endoscope apparatus strongly requires acquiring a taken higher-definition image or taking an image at a high frame rate, illumination light having high intensity is required. For this reason, as in JP2011-72424A, a reflective film having high reflectance, which is formed of a metal film made of silver, aluminum, or the like, is provided around the fluorescent body to effectively use light, which is excited and emitted, as illumination light. Further, to lessen the burden to a patient or the like, it is preferable that the diameter of the insertion section of the endoscope be as small as possible. However, since the outer diameter of the illumination unit should be increased to obtain illumination light having high intensity, there is a problem in that the diameter of the insertion section of the endoscope is increased.

The invention has been made in consideration of the above-mentioned problem, and an object of the invention is to provide an illumination unit for an endoscope and an endoscope that obtain irradiation light having high intensity while suppressing an increase in the diameter of an insertion section of an endoscope.

The invention provides an illumination unit which is mounted on a tip portion of an insertion section of an endoscope. The illumination unit includes: an optical fiber that guides laser light emitted from a light source to a tip portion thereof and emits the laser light; a fluorescent body that is excited by the laser light emitted from the optical fiber and emits fluorescent light; a ferrule that includes a fluorescent body-holding portion holding the fluorescent body and formed at one end thereof, communicates with the fluorescent body-holding portion, and includes an insertion hole into which the optical fiber is inserted and which is formed at the other end thereof; a sleeve that is formed in the shape of a cylinder and holds the ferrule in the cylinder; a protective cover that is mounted on one end of the sleeve so as to cover the fluorescent body held by the ferrule held in the sleeve and transmits light emitted from the fluorescent body; a first sealing portion that seals the protective cover and the sleeve; and a second sealing portion that seals the other ends of the sleeve and the ferrule. When the fluorescent body is formed in a substantially columnar shape, an emission diameter of the fluorescent body is denoted by D1, a thickness of the protective cover is denoted by t1, and an effective diameter of the protective cover is denoted by D2, "0.7 mm≤D1≤0.9 mm", "0.4 mm≤t1≤0.59 mm", and "1.3 mm≤D2≤1.5 mm" are satisfied. Meanwhile, when the amount of light generated in a range of the emission diameter D1 is denoted by B1 and the amount of light generated in a range of the effective diameter D2 is denoted by B2, it is preferable that percentage of light emission efficiency which is calculated by (B2/B1) ×100 be 90% or greater. Further, an endoscope of the invention includes the illumination unit.

According to the invention, when the fluorescent body is formed in a substantially columnar shape, an emission diameter of the fluorescent body is denoted by D1, and an effective diameter of the protective cover is denoted by D2, and a thickness of the protective cover is denoted by t1, it is possible to regulate the thickness of the protective cover without the reduction of the amount of generated light and to provide an illumination unit, which is compact and has excellent protection strength, by setting the emission diameter D1 so as to satisfy "0.7 mm≤D1≤0.9 mm", setting the thickness t1 of the protective cover so as to satisfy "0.4 mm≤t1≤0.59 mm", and setting the effective diameter D2 of the protective cover so as to satisfy "1.3 mm≤D2≤1.5 mm".

### BRIEF DESCRIPTION OF THE DRAWINGS

Fig. 1 is a cross-sectional view showing an illumination unit of the invention.
Fig. 2 is an exploded perspective view of the illumination unit.
Fig. 3 is a cross-sectional view showing the shapes and dimensions of a protective cover, a fluorescent body, a metal reflective film, and a ferrule.
Fig. 4 is a graph showing the light distribution of the fluorescent body.
Fig. 5 is a view showing an example of the disposition of sensors that measure the light distribution of the fluorescent body.
Fig. 6 is a view showing an example of the disposition of sensors that measure the light distribution of the fluorescent body including the protective cover.
Fig. 7 is a graph showing a relationship between a thickness t1 of the protective cover and light emission efficiency while an emission diameter D1 of the fluorescent body is changed to S1, S2, and S3 in three stages when an effective diameter D2 of the protective cover is set to 1.3 mm.
Fig. 8 is a graph showing a relationship between an effective diameter D2 of the protective cover and light emission efficiency while the emission diameter D1 of the fluorescent body is changed to S4, S5, and S6 in three stages when the thickness t1 of the protective cover is set to 0.59 mm.
Fig. 9 is a cross-sectional view showing a second embodiment in which the shapes of the fluorescent body and the metal reflective film are changed.
Fig. 10 is a cross-sectional view showing a third embodiment in which the shapes of the fluorescent body and the metal reflective film are changed.
Fig. 11 is a perspective view showing the overall appearance of an electronic endoscope system of the invention.
Fig. 12 is a cross-sectional view showing a tip portion of an insertion section of an electronic endoscope.
Fig. 13 is a front view of a tip of the insertion section of the electronic endoscope.
Fig. 14 is a block diagram showing the electrical configuration of the electronic endoscope system.

### DESCRIPTION OF THE PREFERRED EMBODIMENTS

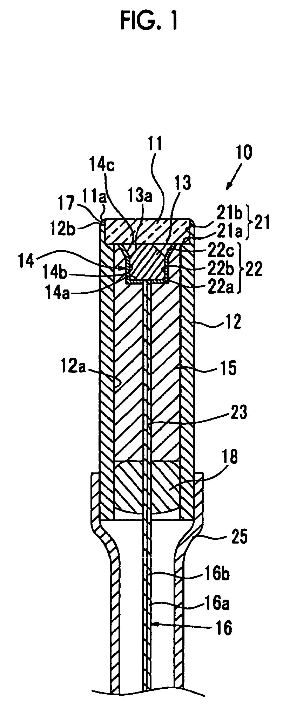

As shown in Fig. 1, an illumination unit 10 of the invention includes a protective cover 11, a sleeve 12, a fluorescent body 13, a metal reflective film 14, a ferrule 15, and an optical fiber 16 in this order from a tip thereof. A first sealing portion 17 is formed between the protective cover 11 and an upper end (one end) of the sleeve 12, and a second sealing portion 18 is formed between a lower end (the other end) of the sleeve 12 and the ferrule 15. The fluorescent body 13 and the metal reflective film 14 are sealed in the sleeve 12 by these sealing portions 17 and 18.

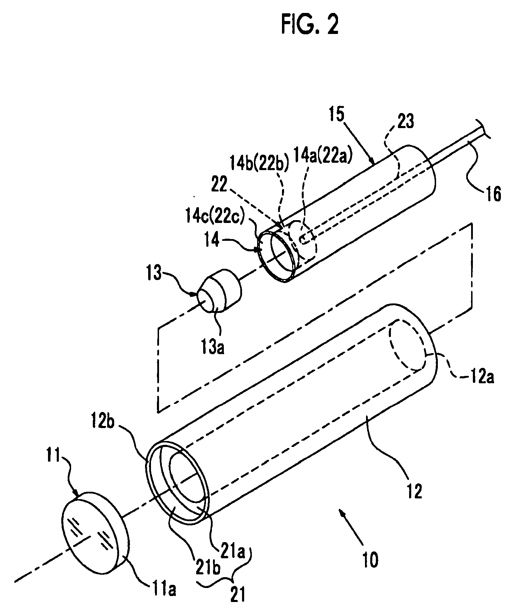

As shown in Fig. 2, the sleeve 12 is formed in the shape of a cylinder having an inner peripheral surface 12a, and a cover receiving portion 21 on which the protective cover 11 is mounted is formed at one end of the sleeve 12. The sleeve 12 is made of a hard material, such as stainless steel, nickel, copper, a copper-tungsten alloy, a copper-molybdenum composite material, or phosphor bronze, carbon, or the like. The cover receiving portion 21 is formed by cutting out the inner peripheral surface 12a of the sleeve 12 in the shape of a step, and includes a stepped surface 21a and an inner peripheral surface 21b. The protective cover 11 is made of, for example, sapphire glass or silica glass and is formed in the shape of a disc. Although not shown, a coat layer that transmits light having a wavelength of, for example, about 445 nm, is formed on each of the surface and the back of the protective cover 11. The thickness of the coat layer is, for example, λ/4 (λ=460nm), and the refractive index of the coat layer is, for example, 1.46.

As shown in Fig. 1, a part of the protective cover 11 protrudes from the cover receiving portion 21 while the protective cover 11 is received in the cover receiving portion 21. A sealant is filled between an outer peripheral surface 11 of a protruding portion of the protective cover 11 and an end face 12b of the sleeve 12, so that a first sealing portion 17 is formed. For example, an epoxy adhesive from which siloxane does not volatilize or the like is preferably used as the sealant.

The ferrule 15 is fitted to the inner peripheral surface 12a of the sleeve 12, and is disposed in the sleeve 12. As shown in Fig. 2, a fluorescent body-holding portion 22 is formed at the tip of the ferrule 15. The fluorescent body-holding portion 22 is formed of a hole that receives the fluorescent body 13, and includes a bottom surface 22a, an inner peripheral surface 22b, and a widened inner peripheral surface 22c.

The metal reflective film 14 is formed on the bottom surface 22a and the inner peripheral surfaces 22b and 22c of the fluorescent body-holding portion 22. The metal reflective film 14 is formed by plating, deposition, sputtering, or the like, and silver or aluminum is used as the material of the metal reflective film 14. In particular, since the reflectance of silver is high, silver is preferably used as the material of the metal reflective film 14. When silver is used, an organic sulfurization preventing layer may be formed on the surface of the silver or bismuth may be added to the silver so that reflectivity and corrosion resistance are improved. Further, if a sufficient thickness can be ensured, an alumina reflective film may be used instead of the metal reflective film 14. Since the metal reflective film 14 is formed on each of the surfaces 22a, 22b, and 22c of the fluorescent body-holding portion 22 as described above, light emitted from the fluorescent body 13 can be repeatedly reflected by the metal reflective film 14. Accordingly, it is possible to emit light toward the protective cover 11 with high light use efficiency. Meanwhile, reference numerals 14a, 14b, and 14c are given to the reflective films of the respective surfaces 22a to 22c so as to correspond to the bottom surface 22a, the inner peripheral surface 22b, and the widened inner peripheral surface 22c.

The fluorescent body 13 is formed substantially in the shape of a column that includes a conical surface 13a at the tip portion thereof. The fluorescent body 13 contains fluorescent materials that form plural kinds of fluorescent bodies (for example, YAG-based fluorescent bodies or fluorescent bodies such as BAM (BaMgAl₁₀O₁₇)) and a resin for fixing and solidification that forms a filler. The plural kinds of fluorescent bodies are excited by absorbing a part of blue laser light, and emit green to yellow light. Accordingly, green to yellow excitation light, which is generated using blue laser light as excitation light, and blue laser light, which is transmitted through the fluorescent body 13 without being absorbed by the fluorescent body 13, are mixed with each other, so that white (pseudo white) illumination light is generated. Since white light having high intensity can be obtained with high light-emitting efficiency when a semiconductor light emitting element is used as an excitation light source as described above, it is possible to easily adjust the intensity of white light and also to suppress the change of the color temperature and the chromaticity of white light to a low level.

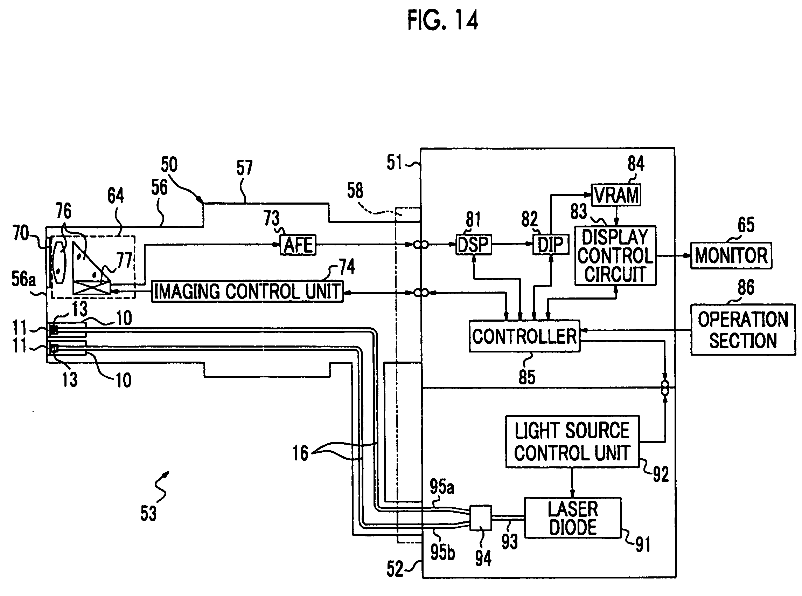

An insertion hole 23 into which the optical fiber 16 is inserted is formed in the ferrule 15 along a center line of the ferrule 15. The insertion hole 23 is opened to the bottom surface 22a of the fluorescent body-holding portion 22. One end of the optical fiber 16 is inserted so as to be exposed to the outside from this opening. Since the other end of the optical fiber 16 is connected to a light source device 52 (see Fig. 14) as described below, the fluorescent body 13 disposed in the fluorescent body-holding portion 22 is irradiated with laser light emitted from the light source device 52. The same metal, the resin, or the like as the material of the sleeve 12 is also used as the material of the ferrule 15. Since it is possible to quickly diffuse heat generated near the fluorescent body 13 when the sleeve 12 and the ferrule 15 are made of the same material having high thermal conductivity as the above-mentioned metal, local heating is prevented.

As shown in Fig. 1, while the ferrule 15 is inserted into the sleeve 12, a sealant is filled inside the inner peripheral surface 12a of a lower end portion of the sleeve 12. As a result, a second sealing portion 18 is formed. The second sealing portion 18 is filled in a gap between the sleeve 12 and the ferrule 15 and a gap between the ferrule 15 and the optical fiber 16, and hermetically seals the ferrule 15 in the sleeve 12. Accordingly, the fluorescent body 13 held by the ferrule 15 and the metal reflective film 14 are isolated from the outside.

A rear end of the sleeve 12 is covered with a protective tube 25. The protective tube 25 protects the optical fiber 16 that is built in the protective tube 25. The optical fiber 16 includes a single mode or multimode fiber body 16a and a protective layer 16b that forms an outer cover.

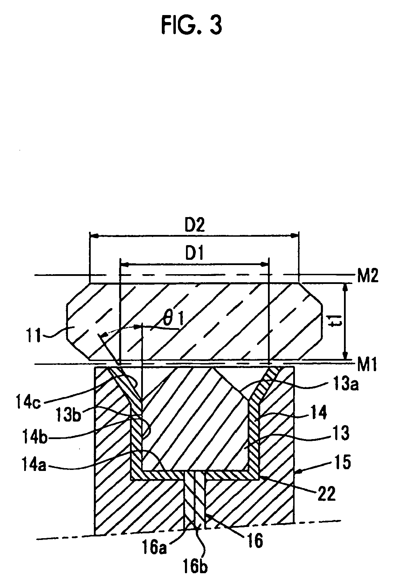

Next, a structure, which improves light emission efficiency, will be described on the basis of a relationship between the protective cover 11 and the fluorescent body 13 with reference to Figs. 3 to 8.

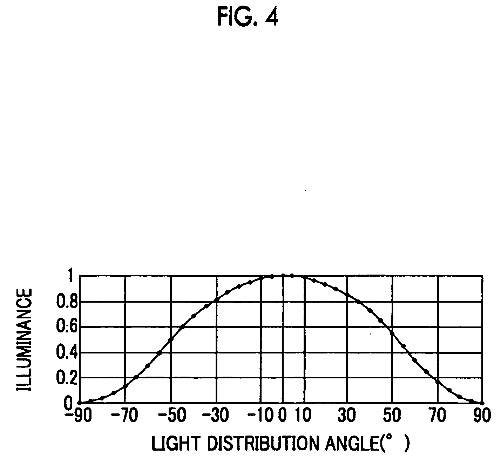

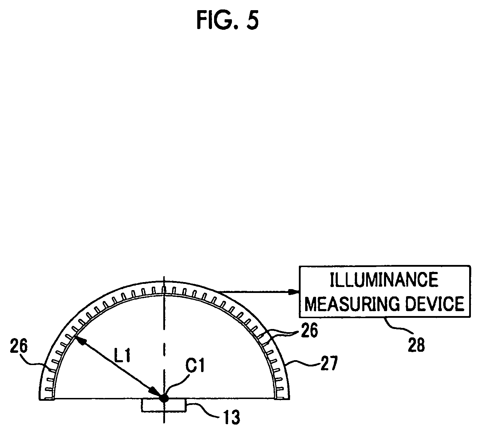

Fig. 3 is a view showing a positional relationship between the protective cover 11 and the fluorescent body 13 while the ferrule 15 is inserted into the sleeve 12 (see Fig. 1). Fig. 4 shows light distribution at a position that is separated from the fluorescent body 13, of which the diameter (emission diameter D1) of a light-emitting surface is 0.8 mm, by a distance of 100 mm, and shows results that are measured in a state shown in Fig. 5. When the metal reflective film 14 is provided around the fluorescent body 13, the emission diameter D1 of the fluorescent body 13 means the maximum diameter of the metal reflective film 14.

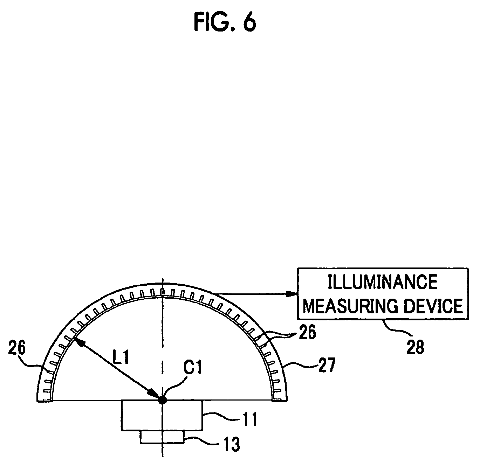

As shown in Figs. 5 and 6, the light distribution of the fluorescent body is measured from the protective cover 11 or the fluorescent body 13, which is a target light source to be measured, by an illuminance measuring device 28 that includes a sensor frame 27. The sensor frame 27 includes optical receivers 26 that are disposed on the circumference of a circle, which has a center point C1 on the target light source to be measured, at an interval of, for example, 5° in a circumferential direction so that a distance L1 is 100 mm. Signals sent from the respective optical receivers 26 of the sensor frame 27 are converted into illuminance by the illuminance measuring device 28, and are displayed on a display of the illuminance measuring device 28 as the light distribution of the fluorescent body shown in Fig. 4. The light distribution of the fluorescent body shown in Fig. 4 is obtained by plotting light distribution angles (°) on a horizontal axis and plotting illuminance on a vertical axis. Meanwhile, illuminance obtained at a light distribution angle of 0° is used as the maximum illuminance "1", and illuminance obtained on the basis of the maximum illuminance is used as the illuminance.

Illuminance B1 obtained at a light-emitting surface M1 of Fig. 3 (illuminance caused by the fluorescent body 13) and illuminance B2 obtained at a light-emitting surface M2 of Fig. 3 (illuminance obtained when light is transmitted through the protective cover 11) are measured by the sensor frame 27 and the illuminance measuring device 28, respectively, and "B2/B1" is obtained as light emission efficiency. The light emission efficiency (B2/B1) is obtained at each thickness when a thickness t1 of the protective cover 11 is in the range of 0.4 to 0.59 mm.

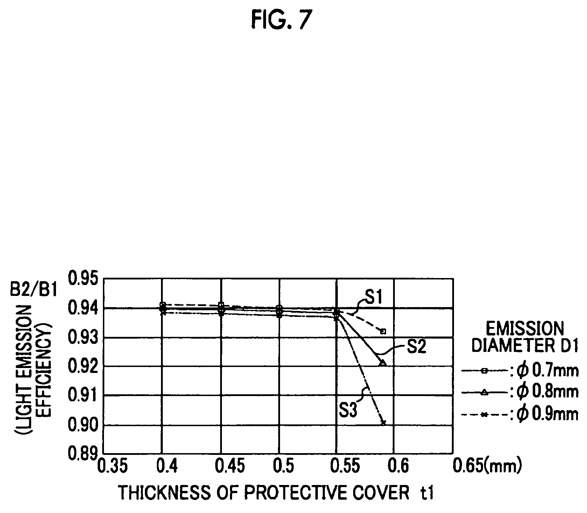

Fig. 7 is a graph showing a relationship between the light emission efficiency (B2/B1) and the thickness t1 of the protective cover 11 while the emission diameter D1 of the fluorescent body 13 is changed to 0.9 mm, 0.8 mm, and 0.7 mm in three stages when the effective diameter D2 of the protective cover 11 is set to 1.3 mm. Meanwhile, the effective diameter D2 means the diameter of a circle that is obtained by excluding a chamfer from the diameter of the protective cover 11 when the protective cover 11 has a circular shape. S1 denotes a characteristic curve when the emission diameter D1 is 0.9 mm, S2 denotes a characteristic curve when the emission diameter D1 is 0.8 mm, and S3 denotes a characteristic curve when the emission diameter D1 is 0.7 mm. As apparent from these characteristic curves S1 to S3, it is found that the light emission efficiency B2/B1 becomes higher as the emission diameter D1 of the fluorescent body 13 is reduced. Further, it is found that the light emission efficiency B2/B1 is reduced as the thickness t1 of the protective cover 11 is increased. Furthermore, it is found that the light emission efficiency is hardly changed in the thickness range of 0.4 mm to 0.55 mm even though the thickness t1 of the protective cover 11 is reduced. Considering the above description overall, it is found that high light emission efficiency of 0.90 or greater can be maintained when the emission diameter is in the range of 0.7 mm to 0.9 mm if the thickness t1 of the protective cover 11 is in the range of 0.4 mm to 0.59 mm. Accordingly, it is possible to suppress the reduction of the light emission efficiency by setting the thickness t1 of the protective cover 11 in the range of 0.4 mm to 0.59 mm.

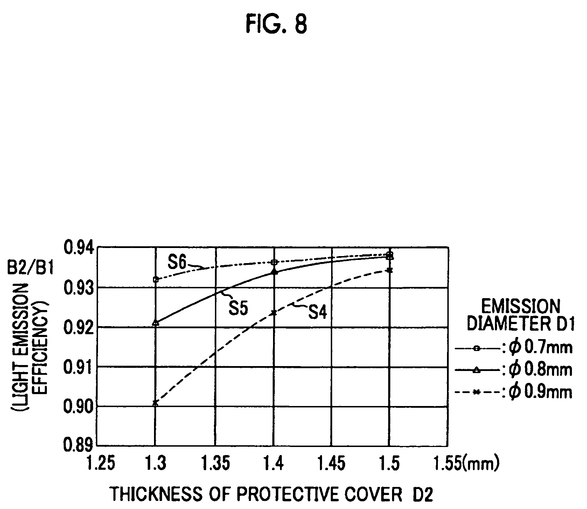

Next, the range of the effective diameter D2 of the protective cover 11, which allows high light-emitting efficiency, is obtained with respect to the emission diameter D1 of the fluorescent body 13. Fig. 8 is a graph showing a relationship between the light emission efficiency (B2/B1) and the effective diameter D2 of the protective cover 11 while the emission diameter D1 is changed to 0.9 mm, 0.8 mm, and 0.7 mm in three stages when the thickness t1 of the protective cover 11 is set to 0.59 mm. S4 denotes a characteristic curve when the emission diameter D1 is 0.9 mm, S5 denotes a characteristic curve when the emission diameter D1 is 0.8 mm, and S6 denotes a characteristic curve when the emission diameter D1 is 0.7 mm. As apparent from these characteristic curves S4 to S6, it is found that the light emission efficiency B2/B1 is reduced as the effective diameter D2 of the protective cover is reduced. Further, it is found that the light emission efficiency is hardly changed when the effective diameter D2 of the protective cover is close to 1.5 mm. Furthermore, it is found that the light emission efficiency is reduced to about 0.9 if the effective diameter D2 of the protective cover is smaller than 1.3 mm when the emission diameter D1 is 0.9 mm.

Overall considering the above description, it is found that percentage of light emission efficiency of 90% or greater is obtained at any thickness t1 when the effective diameter D2 of the protective cover satisfies "1.3 mm≤D2≤1.5 mm" if the thickness t1 of the protective cover 11 is 0.59 mm or less. Further, when the thickness t1 of the protective cover 11 is increased, the light emission efficiency is reduced as also apparent from Fig. 7. Accordingly, if the thickness t1 exceeds 0.59 mm, from the relationship of Fig. 7, the light emission efficiency is reduced to about 0.9 when the emission diameter D1 of the fluorescent body is 0.9 mm. For this reason, it is not preferable that the thickness t1 exceed 0.59 mm. Furthermore, since the thickness of the protective cover 11 is reduced when the thickness t1 of the protective cover 11 is smaller than 0.59 mm, the light emission efficiency is increased. Accordingly, even though the effective diameter D2 of the protective cover is obtained when the thickness t1 is 0.59 mm or greater, there is no particular problem.

The protective cover 11, which is used for the above-mentioned measurement, has a refractive index nd of 1.883 (a refractive index with respect to a line d), a refractive index ne of 1.88813 (a refractive index with respect to a line e), a variance ud of 40.8 (a variance with respect to a line d), and a variance ue of 40.6 (a variance with respect to a line e). The measured data are obtained by the actual measurement that is performed while the emission diameter D1 and the thickness t1 of the cover 11 are changed. Meanwhile, data, which is obtained by a simulation, may be used instead of data that are actually measured using measurement units shown in Figs. 5 and 6. In this case, measured data may be reproduced and simulation calculation may be performed by using LightTools (registered trademark), which is manufactured by Synopsys, Inc., as a simulation application and using a diffusing surface of, for example, cos1.37 squared as the definition of, for example, a light source on the simulation. The protective cover 11 is provided with the coat layer to improve the light emission efficiency. The thickness of the coat layer is, for example, λ/4 (λ=460 nm), and the refractive index of the coat layer is, for example, 1.46.

From the point of view of the improvement of the light emission efficiency, the effective diameter D2 of the protective cover 11 is not limited to an upper limit of 1.5 mm. However, when the effective diameter D2 of the protective cover 11 is set to a value exceeding 1.5 mm, the diameter of the illumination unit 10 is increased and the diameter of the endoscope insertion section is also increased accordingly. Accordingly, it is not preferable that the effective diameter D2 of the protective cover 11 be set to a value exceeding 1.5 mm. Further, if the effective diameter D2 of the protective cover 11 is set to a value smaller than 1.3 mm, as also found from Fig. 8, the light emission efficiency is reduced to 0.9 or less or the amount of light emitted from the protective cover is also reduced due to the reduction of the diameter of the effective diameter D2 of the fluorescent body 13 particularly when the emission diameter D1 is 0.9 mm. Accordingly, it is not preferable that the effective diameter D2 of the protective cover 11 be set to a value smaller than 1.3 mm.

Furthermore, when the metal reflective film 14 includes a widened reflective film 14c that is gradually widened outward as shown in Figs. 1 and 2, the amount of illumination light can be increased due to the increase of the amount of light reflected by the widened reflective film 14c. Accordingly, it is preferable that the metal reflective film 14 includes a widened reflective film 14c. Meanwhile, the fluorescent body 13 may be formed so as to include the conical surface 13a that is formed by cutting the outer peripheral surface portion of the fluorescent body 13, which faces the widened reflective film 14c, in a conical shape according to the widened reflective film 14c. Since it is also possible to increase the light-emitting area, which can be used as illumination light source, by this structure, a total amount of illumination light may be increased. It is preferable that a widening angle θ1 of the widened reflective film 14c with respect to a holding hole-inner peripheral surface 13b be in the range of 15° to 60°. In this case, since light, which is emitted from the conical surface 13a or the outer peripheral surface of the fluorescent body 13, also can be effectively used as illumination light, efficiency is improved.

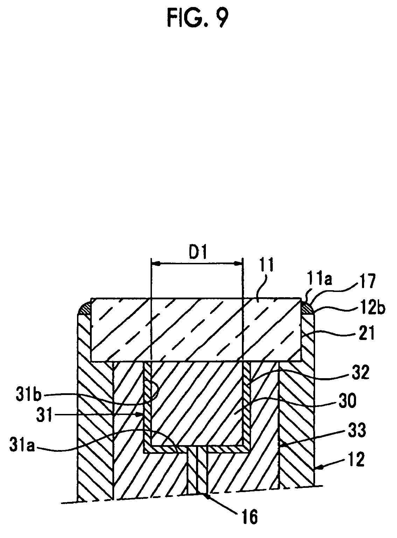

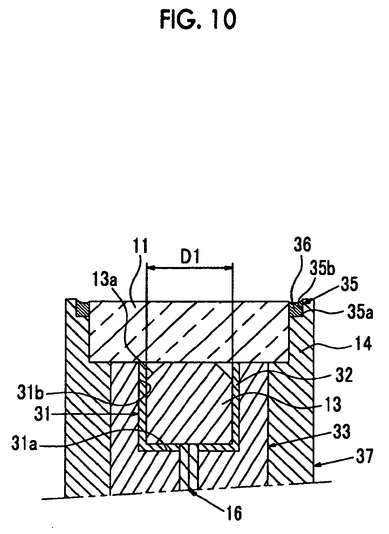

A part of the outer peripheral surface of the fluorescent body 13 has been formed of the conical surface 13a according to the widened reflective film 14c in the above-mentioned embodiment, but the fluorescent body 30 may be formed instead in a columnar shape as shown in Fig. 9. In this case, the widened reflective film 14c may not be formed on a ferrule 33 and a metal reflective film 32 may be formed on only a bottom surface 31a and an outer peripheral surface 31b of a fluorescent body-holding portion 31. Alternatively, the columnar fluorescent body 30 shown in Fig. 9 may be used together with the fluorescent body-holding portion 22 and the metal reflective film 14 shown in Fig. 1. In addition, as shown in Fig. 10, a fluorescent body 13, which includes a conical surface 13a at the tip thereof as shown in Fig. 1, may be used together with the ferrule 33 that includes the fluorescent body-holding portion 31 shown in Fig. 9. Meanwhile, in the respective embodiments, the same members are denoted by the same reference numerals and the repeated description thereof is omitted.

As shown in Figs. 1 and 9, the first sealing portion 17 is made of a sealant that is filled between a part of the outer peripheral surface 11a of the protective cover 11 and a part of the end face 12b of the sleeve 12. However, instead of the first sealing portion 17, as shown in Fig. 9, a sealant receiving step portion 35, which includes a stepped surface 35a and an inner peripheral surface 35b, may be formed on the end face of the sleeve 12 and a sealant may be filled in the sealant receiving step portion 35 to form a first sealing portion 36. In this case, a tip corner of the protective cover 11 is protected at a tip portion of a sleeve 37 without protruding to the outside.

Further, in the above-mentioned embodiment, a gap between the sleeve 12 and the ferrule 15 and a gap between the ferrule 15 and the optical fiber 16 are collectively sealed by the second sealing portion 18 as shown in Fig. 1. However, instead of the second sealing portion 18, a sealant individually seals a gap between the ferrule 15 and the optical fiber 16 and a gap between the outer peripheral surface of the ferrule 15 and the inner peripheral surface of the sleeve 12, so that a second sealing portion 18 may be formed.

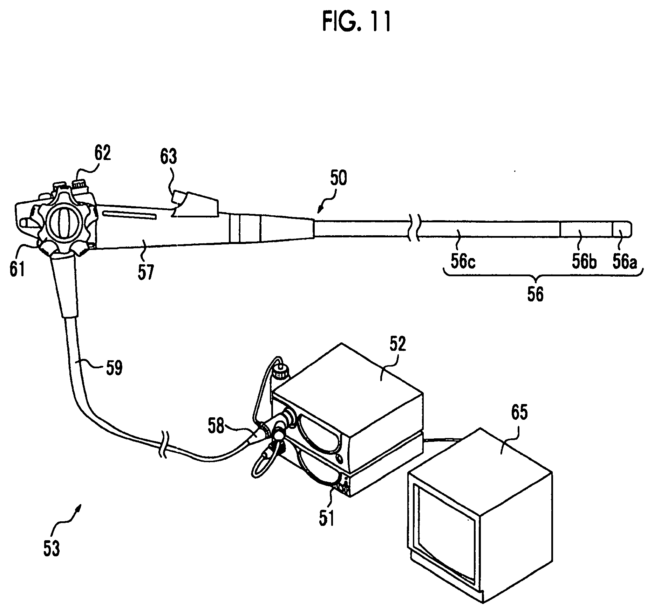

As shown in Figs. 11 to 14, the illumination units 10 of the invention are built in a tip portion 56a of an insertion section of an electronic endoscope 50 while being used. The electronic endoscope 50 is connected to a processor device 51 and the light source device 52, and the electronic endoscope 50, the processor device 51, and the light source device 52 form an electronic endoscope system 53. The electronic endoscope 50 includes a flexible insertion section 56 that is inserted into a patient's body cavity, an operation section 57 that is connected to a base end portion of the insertion section 56, a connector 58 that is connected to the processor device 51 and the light source device 52, and a universal code 59 that connects the operation section 57 to the connector 58.

The insertion section 56 includes a tip portion 56a, a bendable portion 56b, and a flexible tube portion 56c in this order from a tip thereof. An imaging unit and the illumination unit 10 of the invention are built in the tip portion 56a. The bendable portion 56b is adapted to be capable of being bent by the operation of a wire. The flexible tube portion 56c has flexibility, and connects the bendable portion 56b to the operation section 57.

The operation section 57 is provided with operation members that are an angle knob 61 for allowing the bendable portion 56b to be bent vertically and laterally and an air supply/water supply button 62 for allowing air or water to be ejected from the tip portion 56a. Further, the operation section 57 is provided with a forceps port 63 that allows a treatment tool, such as an electrical scalpel, to be inserted into a forceps channel (not shown).

The processor device 51 is electrically connected to the light source device 52, and generally controls the operation of the electronic endoscope system 53. The processor device 51 drives an imaging unit 64 by supplying power to the electronic endoscope 50 through the universal code 59 and a transmission cable that is inserted into the insertion section 56. Further, the processor device 51 acquires an imaging signal that is output from the imaging unit 64 through the transmission cable, and generates image data by performing various kinds of image processing. The image data, which are generated by the processor device 51, are displayed on a monitor 65 as observed images.

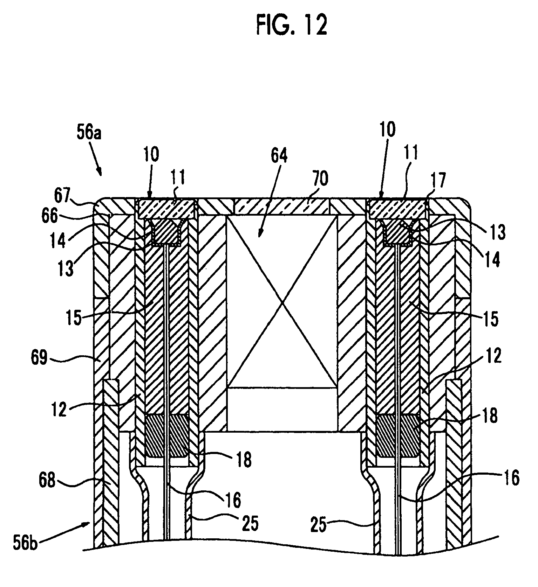

As shown in Fig. 12, the tip portion 56a includes a tip hard portion 66 and a tip-protection cap 67 that is mounted on the tip of the tip hard portion 66. The tip hard portion 66 is made of, for example, stainless steel, and a plurality of through holes are formed at the tip hard portion 66 along a longitudinal direction. Various components, such as two illumination units 10, the imaging unit 64, a forceps channel, and an air supply/water supply channel (not shown), are mounted in the respective through holes of the tip hard portion 66. A rear end of the tip hard portion 66 is connected to a bendable piece 68 of a tip that forms the bendable portion 56b. Further, the outer periphery of the tip hard portion 66 is covered with an outer tube 69.

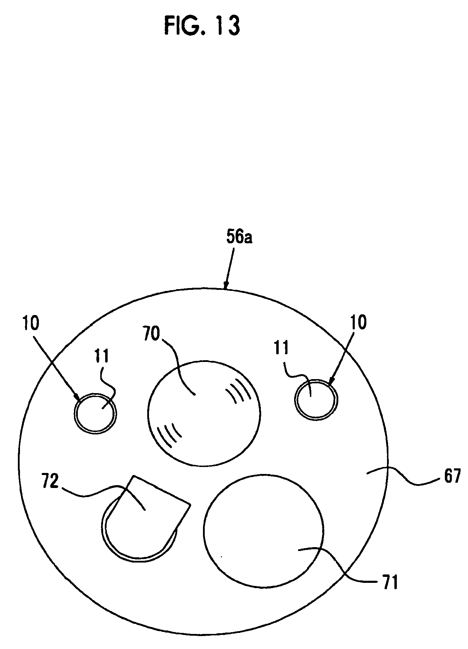

The tip-protection cap 67 is made of rubber or an elastomer made of a resin, and through holes are formed in the tip-protection cap 67 at positions corresponding to various components that are held by the tip hard portion 66. As shown in Fig. 13, an observation window 70, the two (a pair of) illumination units 10, a forceps outlet 71, an air supply/water supply nozzle 72, and the like are exposed to the outside through the respective holes of the tip-protection cap 67. The pair of illumination units 10 are disposed at positions that are symmetrical to each other with the observation window 70 interposed therebetween.

As shown in Fig. 14, the electronic endoscope 50 includes the imaging unit 64 and the two illumination units 10 that are provided at the tip portion 56a, and an AFE (analog signal processing circuit) 73 and an imaging control unit 74 that are provided at the operation section 57.

The imaging unit 64 is disposed in the observation window 70, and includes an imaging optical system 76 that is formed of a lens group and a prism and a CCD 77 in which an image inside the body cavity is formed on an imaging plane by the imaging optical system 76. The CCD 77 accumulates signal charges by photoelectrically converting the image inside a subject, which is formed on the imaging plane, and outputs the accumulated signal charges as imaging signals. The output imaging signals are sent to the AFE 73. The AFE 73 includes a correlated double sampling (CDS) circuit, an automatic gain control (AGC) circuit, an A/D converter, and the like (of which all are not shown). The CDS performs correlated double sampling processing on the imaging signals that are output from the CCD 77, and removes noise that is generated by the drive of the CCD 77. The AGC amplifies the imaging signals from which noise has been removed by the CDS.

When the electronic endoscope 50 and the processor device 51 are connected to each other, the imaging control unit 74 is connected to a controller 85 provided in the processor device 51. When receiving an instruction from the controller 85, the imaging control unit 74 sends a drive signal to the CCD 77. The CCD 77 outputs imaging signals to the AFE 73 at a predetermined frame rate on the basis of the drive signal that is sent from the imaging control unit 74.

The optical fibers 16 of the illumination units 10 guide blue laser light, which is supplied from the light source device 52, and emits the blue laser light to the fluorescent bodies 13 that are provided on emission end sides thereof. The fluorescent bodies 13 are excited by absorbing a part of the blue laser light emitted from the optical fibers 16, and emit green to yellow light. For this reason, blue light, which is transmitted through the fluorescent bodies 13 while being diffused in the fluorescent bodies 13, and green to yellow fluorescent light, which is excited and emitted from the fluorescent bodies 13, are mixed to each other in the illumination units 10, so that white (pseudo white) illumination light is formed. The irradiation range of the illumination light is substantially equal to or larger than the range of an image that is taken by the electronic endoscope 50, and the entire observed image is substantially uniformly irradiated with the illumination light.

The processor device 51 includes a digital signal processing circuit (DSP) 81, a digital image processing circuit (DIP) 82, a display control circuit 83, a VRAM 84, the controller 85, an operation section 86, and the like.

The controller 85 generally controls the operation of the entire processor device 51. The DSP 81 generates image data by performing various kinds of signal processing, such as color separation, color interpolation, gain correction, white balance adjustment, and gamma correction, on the imaging signals that are output from the AFE 73 of the electronic endoscope 50. The image data, which are generated by the DSP 81, are input to a working memory of the DIP 82. Further, the DSP 81 generates data for ALC control, which are required for the automatic light control (ALC control) of the amount of illumination light, such as an average luminance value that is an average of luminance values of the respective pixels of the generated image data, and inputs the data for ALC control to the controller 85.

The DIP 82 performs various kinds of image processing, such as electronic variable magnification, color enhancement processing, and edge enhancement processing, on the image data that are generated by the DSP 81. The image data, which has been subjected to the various kinds of image processing performed by the DIP 82, are temporarily stored in the VRAM 84 as observed images, and are then input to the display control circuit 83. The display control circuit 83 selects and acquires an observed image from the VRAM 84, and displays the observed image on the monitor 65.

The operation section 86 is formed of well-known input devices, such as an operation panel, a mouse, and a keyboard, which are provided in a housing of the processor device 51. The controller 85 operates the respective sections of the electronic endoscope system 53 according to an operation signal that is sent from the operation section 86 or the operation section 57 of the electronic endoscope 50.

The light source device 52 includes a laser diode (LD) 91 as a laser light source and a light source control unit 92. The LD 91 is a light source that emits blue laser light having a center wavelength of 445 nm, and the blue laser light is guided to an optical fiber 93 through a condensing lens (not shown) and the like. The optical fiber 93 is connected to two optical fibers 95a and 95b through a branch coupler 94. The optical fibers 95a and 95b are connected to the optical fibers 16 of the electronic endoscope 50 through a connector 58. For this reason, blue laser light emitted from the LD 91 enters the fluorescent bodies 13 that form the illumination units 10. Further, when the blue laser light enters the fluorescent bodies, the blue laser light is mixed to the green to yellow fluorescent light, which is excited and emitted from the fluorescent bodies 13, and a portion to be observed is irradiated with the mixed light as white illumination light.

The light source control unit 92 adjusts the turn-on/turn-off timing of the LD 91 according to an adjustment signal or a synchronous signal that is input from the controller 85 of the processor device 51. Further, the light source control unit 92 adjusts the amount of illumination light, which irradiates the portion to be observed, by communicating with the controller 85 and adjusting the amount of light generated from the LD 91. The control of the amount of illumination light, which is performed by the light source control unit 92, is ALC (automatic light control) control that automatically adjusts the amount of illumination light according to the brightness or the like of the observed image having been taken, and is performed on the basis of the data for ALC control that are generated by the DSP 81.

It is possible to illuminate the portion to be observed with the illumination light having high intensity by using the illumination unit 10 of the invention as described above. Accordingly, it is possible to acquire a taken high-definition image or to take an image at a high frame rate by the imaging unit.

The electronic endoscope, which observes an image of the state of the portion to be observed taken by an imaging element, has been described by way of example in the embodiments. However, the invention is not limited thereto, and also may be applied to an endoscope that observes the state of a portion to be observed by an optical image guide. Further, the endoscope including two illumination optical system units has been described by way of example in the embodiments. However, the invention is not limited thereto, and also may be applied to an endoscope including one illumination optical system unit or an endoscope including three or more illumination optical system units.

## Claims

1. An illumination unit (10) for an endoscope which is mounted on a tip portion of an insertion section of an endoscope, the illumination unit comprising:
an optical fiber (16) that guides laser light emitted from a light source to a tip portion thereof and emits the laser light;
a fluorescent body (13) that is excited by the laser light emitted from the optical fiber and emits fluorescent light;
a ferrule (15) that includes a fluorescent body-holding portion holding the fluorescent body and formed at one end thereof, and includes an insertion hole that communicates with the fluorescent body-holding portion, into which the optical fiber is inserted and which is formed at the other end thereof;
a sleeve (12) that is formed in the shape of a cylinder and holds the ferrule therein;
a protective cover (11) that is mounted on one end of the sleeve so as to cover the fluorescent body held by the ferrule held in the sleeve and transmits light emitted from the fluorescent body;
a first sealing portion (17) that seals the protective cover and the sleeve; and
a second sealing portion (18) that seals the other ends of the sleeve and the ferrule,
**characterized in that** when the fluorescent body is formed in a substantially columnar shape, an emission diameter of the fluorescent body is denoted by D1, a thickness of the protective cover is denoted by t1, and an effective diameter of the protective cover is denoted by D2, "0.7 mm≤D1≤0.9 mm", "0.4 mm≤t1≤0.59 mm", and "1.3 mm≤D2≤1.5 mm" are satisfied.

2. The illumination unit for an endoscope according to claim 1,
wherein when the amount of light generated in a range of the emission diameter D1 is denoted by B1 and the amount of light generated in a range of the effective diameter D2 is denoted by B2, the percentage of light emission efficiency which is calculated by (B2/B1)×100 is 90% or greater.

3. The illumination unit for an endoscope according to claim 1 or 2,
wherein the ferrule includes a light reflection film that is formed on an inner peripheral surface of the fluorescent body-holding portion,
the fluorescent body-holding portion includes a holding hole that holds the fluorescent body, and a widened reflective film that is connected to an inner wall surface of the holding hole and is gradually widened at the one end side of the outer peripheral surface of the fluorescent body, and
the emission diameter D1 of the fluorescent body is a maximum opening diameter of the widened reflective film.

4. An endoscope comprising:
the illumination unit for an endoscope according to any one of claims 1 to 3.

## Patentansprüche

1. Beleuchtungseinheit (10) für ein Endoskop, angebracht an einer Spitze eines Einführabschnitts eines Endoskops, wobei die Beleuchtungseinheit umfasst:
eine Lichtleitfaser (16), die von einer Lichtquelle emittiertes Laserlicht zu ihrer Spitze führt und das Laserlicht emittiert;
einen Fluoreszenzkörper, der von dem von der Lichtleitfaser emittierten Laserlicht angeregt wird und Fluoreszenzlicht emittiert;
eine Zwinge (15), die einen Fluoreszenzkörper-Halteabschnitt enthält, welcher den Fluoreszenzkörper hält und an dessen einem Ende ausgebildet ist, und die ein Einführloch enthält, welches mit dem Fluoreszenzkörper-Haltekörper kommuniziert, in welches die Lichtleitfaser eingeführt ist, und welches an deren anderem Ende ausgebildet ist;
eine Hülse (12), die in Form eines Zylinders ausgebildet ist und in sich die Zwinge trägt;
eine Schutzabdeckung (11), die an einem Ende der Hülse angebracht ist, um den von der in der Hülse gehaltenen Zwinge gehaltenen Fluoreszenzkörper abzudecken, und die von dem Fluoreszenzkörper emittiertes Licht durchlässt;
einen ersten Dichtungsabschnitt (17), der die Schutzabdeckung und die Hülse abdichtet; und
einen zweiten Dichtungsabschnitt (18), der die anderen Enden der Hülse und der Zwinge abdichtet,
**dadurch gekennzeichnet, dass**, wenn der Fluoreszenzkörper in einer wesentlichen säulenförmigen Form ausgebildet ist, ein Emissionsdurchmesser des Fluoreszenzkörpers mit D1 bezeichnet ist, eine Dicke der Schutzabdeckung mit t1 bezeichnet ist, und ein effektiver Durchmesser der Schutzabdeckung mit D2 bezeichnet ist, "0,7 mm≤D1≤0,9 mm", "0,4 mm≤t1≤0,59 mm", und "1,3 mm≤D2≤1,5 mm" erfüllt sind.

2. Beleuchtungseinheit für ein Endoskop nach Anspruch 1,
bei dem, wenn die im Bereich des Emissionsdurchmessers D1 erzeugte Lichtmenge mit B1 bezeichnet wird und die in dem Bereich des effektiven Durchmessers D2 erzeugte Lichtmenge mit B2 bezeichnet wird, der durch (B2/B1)×100 berechnete Prozentsatz des Lichtemissionswirkungsgrads 90 % oder größer ist.

3. Beleuchtungseinheit für ein Endoskop nach Anspruch 1 oder 2,
bei der die Zwinge eine Lichtreflexionsschicht enthält, die an der Innenumfangsfläche des Fluoreszenzkörper-Halteabschnitts gebildet ist,
der Fluoreszenzkörper-Halteabschnitt ein Halteloch enthält, welches den Fluoreszenzkörper hält, und einen erweiterten Reflexionsfilm enthält, der mit einer Innenwandfläche des Haltelochs verbunden ist und an der einen Endseite der Außenumfangsfläche des Fluoreszenzkörpers allmählich aufgeweitet ist, und
der Emissionsdurchmesser D1 des Fluoreszenzkörpers ein maximaler Öffnungsdurchmesser des erweiterten Reflexionsfilms ist.

4. Endoskop, umfassend:
die Beleuchtungseinheit für ein Endoskop nach einem der Ansprüche 1 bis 3.

## Revendications

1. Unité d'éclairage (10) destinée à un endoscope monté sur une portion de bout d'une section d'introduction d'un endoscope, l'unité d'éclairage, comprenant :
une fibre optique (16) qui guide une lumière laser émise d'une source de lumière vers une partie de bout de celle-ci et émet la lumière laser ;
un corps fluorescent (13) qui est excité par la lumière laser émise à partir de la fibre optique et émet une lumière fluorescente ;
une virole (15) qui inclut une portion de retenue de corps fluorescent retenant le corps fluorescent et formée au niveau d'une extrémité de celui-ci, et qui inclut un trou d'introduction qui communique avec la portion de retenue de corps fluorescent, dans lequel la fibre optique est introduite et qui est formé sur l'autre extrémité de celui-ci ;
un manchon (12) qui est façonné sous la forme d'un cylindre et qui retient la virole;
un couvercle protecteur (11) qui est monté sur une extrémité du manchon de manière à recouvrir le corps fluorescent retenu par la virole dans le manchon et qui transmet la lumière émise à partir du corps fluorescent ;
une première portion d'étanchéité (17) qui scelle le couvercle protecteur et le manchon, et
une seconde portion d'étanchéité (18) qui scelle les autres extrémités du manchon et la virole,
**caractérisée en ce que** lorsque le corps fluorescent est façonné suivant une forme en grande partie de colonne, un diamètre d'émission du corps fluorescent est indiqué par D1, une épaisseur du couvercle protecteur est indiquée par t1, et un diamètre effectif du couvercle protecteur est indiqué par D2, et les expressions "0,7 mm≤D1≤0,9 mm",
"0,4 mm≤t1≤0,59 mm", et "1,3 mm≤D2≤1,5 mm" sont satisfaites.

2. Unité d'éclairage destinée à un endoscope selon la revendication 1,
dans laquelle lorsque la quantité de lumière générée dans un intervalle du diamètre d'émission D1 est indiquée par B1, et la quantité de lumière générée dans un intervalle du diamètre effectif D2 est indiquée par B2, le pourcentage d'efficacité de l'émission lumineuse, qui est calculé par (B2/B1) x 100, est supérieur ou égal à 90%.

3. Unité d'éclairage destinée à un endoscope selon la revendication 1 ou 2,
dans laquelle la virole inclut un film reflétant la lumière qui est formé sur une surface périphérique intérieure de la portion de retenue de corps fluorescent,
la portion de retenue de corps fluorescent inclut un trou de retenue qui retient le corps fluorescent, et un film réflecteur élargi qui est relié à une surface de paroi intérieure du trou de retenue et est progressivement élargi au niveau d'un côté d'extrémité de la surface périphérique extérieure du corps fluorescent, et
le diamètre d'émission D1 du corps fluorescent est un diamètre d'ouverture maximal du film réflecteur élargi.

4. Endoscope comprenant :
l'unité d'éclairage destinée à un endoscope selon l'une quelconque des revendications 1 à 3.
